# DEMANDE DE BREVET EUROPEEN

(11) **EP 1 535 596 A1**
(43) Date de publication de la demande: **01.06.2005**
(21) Numéro de dépôt: 04292536.2
(22) Date de dépôt: 26.10.2004
(51) Int. Cl.: A61K 7/09

(54) **Composition de mise en forme permanente des cheveux comprenant au moins une amine secondaire ou tertiaire**

(30) Priorité: 18.11.2003 FR 0350854
(71) Demandeur: L'OREAL, 75008 Paris (FR)
(72) Inventeur: Malle, Gérard, 77580 Villiers sur Morin (FR); Lavergne, Damien, 75015 Paris (FR); Radisson, Xavier, 75014 Paris (FR)
(74) Mandataire: Bourdeau, Françoise

(57) **Abrégé**

L'invention a pour objet une composition cosmétique de mise en forme permanente des fibres kératiniques prête à l'emploi contenant, en tant qu'agent actif de mise en forme permanente, une amine secondaire ou tertiaire. Elle vise également un kit contenant des compartiments à mettre en contact pour former la composition prête à l'emploi, ainsi qu'un procédé mettant en oeuvre cette composition.

## Description

L'invention a pour objet une composition cosmétique de mise en forme permanente des fibres kératiniques prête à l'emploi contenant, en tant qu'agent actif de mise en forme permanente, une amine secondaire ou tertiaire. Elle vise également un kit contenant des compartiments à mettre en contact pour former la composition prête à l'emploi, ainsi qu'un procédé mettant en oeuvre cette composition.

Par *"fibres kératiniques",* on entend, selon l'invention, des fibres d'origine humaine ou animale telles que les cheveux, les poils, les cils, la laine, l'angora, le cachemire ou la fourrure. Bien que l'invention ne soit pas limitée à des fibres kératiniques particulières, il sera néanmoins fait plus particulièrement référence aux cheveux.

Le terme *"mise en forme permanente"* englobe, selon l'invention, le frisage, la permanente, la mise en plis de cheveux caucasiens, asiatiques ou nord-africains.

Le terme *"amine secondaire ou tertiaire"* englobe les amines et leurs sels organiques ou minéraux ne contenant pas dans leur formule chimique d'ions hydroxydes.

Par *"amine secondaire ou tertiaire",* on entend, selon l'invention, un composé capable d'accepter un proton et dérivant de la substitution de deux ou trois atomes d'hydrogène de l'ammoniac par des radicaux carbonés simplement liés à l'atome d'azote.

Dans ce texte, l'expression *"compris entre x % et y %"* signifie allant de x à y %, les bornes x et y étant comprises.

Deux techniques sont utilisées pour obtenir une déformation permanente des cheveux. Elles sont basées sur une rupture des liaisons disulfures présentes dans la kératine (cystine) :
- la première consiste, dans un premier temps à réaliser cette ouverture des liaisons disulfures à l'aide d'une composition contenant un agent réducteur, puis après avoir, de préférence, rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur, de façon à donner à la chevelure la forme recherchée. Cette technique permet indifféremment de réaliser, soit la mise en forme permanente des cheveux, soit leur défrisage ou leur décrêpage ou leur lissage.
- La seconde consiste à effectuer une opération dite de lanthionisation, à l'aide d'une composition contenant une base appartenant à la famille des hydroxydes. Elle conduit à remplacer des liaisons disulfures (-CH2-S-S-CH2-) par des liaisons lanthionines (-CH2-S-CH2-). Cette opération de lanthionisation fait intervenir deux réactions chimiques consécutives :
   ■ La première réaction consiste en une béta-élimination sur la cystine provoquée par un ion hydroxyde, conduisant à la rupture de cette liaison et à la formation de déhydro-alanine. 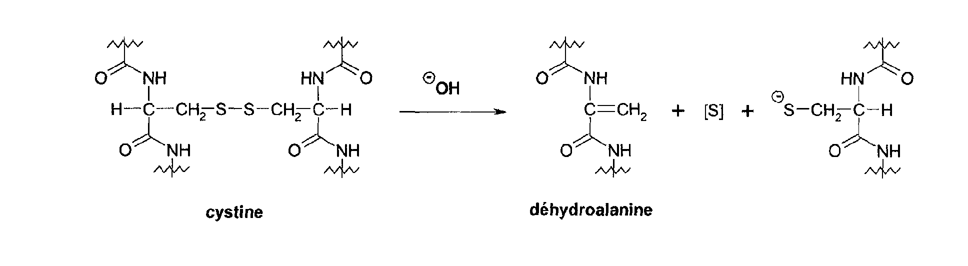
   ■ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine.

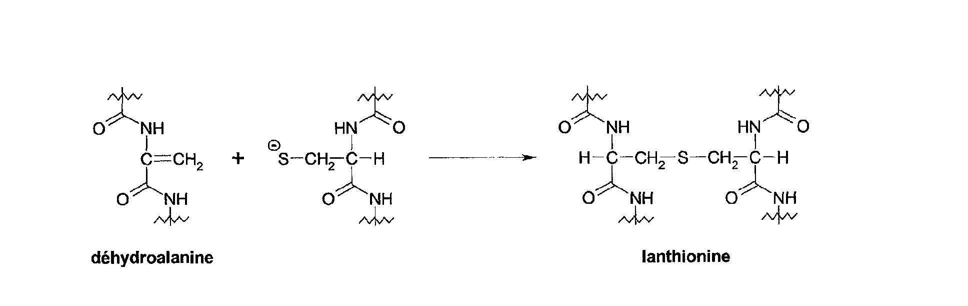

Par rapport à la première technique mettant en oeuvre un agent réducteur, cette technique de lanthionisation ne nécessite pas d'étape de fixation, puisque la formation des ponts lanthionine est irréversible. Elle s'effectue donc en une seule étape et permet indifféremment de réaliser soit l'ondulation des cheveux, soit leur défrisage ou leur décrêpage ou leur lissage. Cependant, elle est principalement utilisée pour le défrisage des cheveux naturellement crépus.

Pour la première technique, les compositions réductrices généralement utilisées pour la première étape d'une opération de permanente ou de défrisage contiennent à titre d'agent réducteur des thiols ou des sulfites ou des bisulfites. Ces agents sont généralement employés en milieu essentiellement aqueux à des concentrations comprises entre 0,5 et 1M pour obtenir une bonne ouverture des liaisons disulfures. Parmi les thiols, ceux couramment utilisés sont l'acide thioglycolique, la cystéamine, le monothioglycolate de glycérol, l'acide thiolactique et la cystéine. L'acide thioglycolique est particulièrement efficace pour réduire les liaisons disulfures de la kératine à pH alcalin, notamment sous forme de thioglycolate d'ammonium, et constitue le produit le plus utilisé en permanente (" hair waving "). On a toutefois constaté que l'acide thioglycolique doit être utilisé en milieu suffisamment basique (en pratique à pH compris entre 8,5 et 9,5) si on veut obtenir une frisure satisfaisante en intensité. Outre l'inconvénient de dégager une odeur désagréable nécessitant l'usage de parfums plus ou moins efficaces pour masquer les odeurs, l'utilisation d'un thiol à pH alcalin conduit également à des dégradations de la fibre et tout particulièrement à l'altération des colorations artificielles.

Les sulfites ou bisulfites sont principalement utilisés pour le défrisage. Ils possèdent des désavantages similaires aux thiols avec une efficacité moindre.

Les thiols et les sulfites (ou bisulfites) présentent en outre l'inconvénient d'avoir une mauvaise stabilité en solution aqueuse.

D'une façon générale, la durabilité des effets de déformations obtenus avec les thiols et les sulfites par réduction des disulfures puis fixation est jugée très inférieure à celle pouvant être obtenue par la technique de lanthionisation.

Pour la deuxième technique, les compositions généralement utilisées pour effectuer la lanthionisation contiennent à titre de base un hydroxyde tel que l'hydroxyde de sodium, l'hydroxyde de guanidinium et l'hydroxyde de lithium. Ces actifs de lanthionisation, permettant d'ouvrir les liaisons disulfures par un mécanisme de béta-élimination, sont généralement employés en émulsion eau-huile à des concentrations comprises entre 0,4 et 0,6M, en les laissant agir généralement 10 à 15 minutes à température ambiante. L'hydroxyde de sodium reste l'agent le plus utilisé. L'hydroxyde de guanidinium est maintenant le composé préféré pour de nombreuses compositions. Ces deux hydroxydes, de sodium et de guanidinium, sont les deux agents principaux utilisés pour le défrisage ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise (durée du traitement plus courte), et une durabilité et une efficacité beaucoup plus importante de la déformation du cheveu.

Cependant, ces hydroxydes présentent l'inconvénient majeur d'être caustiques. Cette causticité affecte le cuir chevelu en provoquant des irritations parfois sévères. On peut y remédier partiellement par l'application préalable sur le scalp d'une crème protectrice grasse souvent appelée " base " ou " crème base ", le mot " base " ici utilisé n'ayant pas la signification d'agent basique au sens chimique. Lorsque la crème protectrice est associée à l'hydroxyde en une seule composition, celle-ci est généralement appelée " no-base ", par opposition à l'appellation ci-dessus. Cette technologie " no-base " est préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

Les formules contenant de l'hydroxyde de sodium sont généralement dénommées en anglais " lye relaxers" et celles n'en contenant pas sont dénommées " no-lye relaxers ".
Les principales formules défrisantes dites " no-lye " mettent en oeuvre l'hydroxyde de guanidinium. L'hydroxyde de guanidinium étant instable, celui-ci est généré extemporanément par le mélange de carbonate de guanidine et d'une source d'hydroxyde très peu soluble telle que l'hydroxyde de calcium. La réaction entre ces deux composés conduit à la formation d'hydroxyde de guanidinium et de carbonate de calcium qui précipite dans la composition. La présence de ce précipité rend le rinçage final des cheveux beaucoup plus difficile et laisse sur les cheveux et le scalp des particules minérales qui lui donnent un toucher rêche et une apparence inesthétique ressemblant aux pellicules. Le succès récent de l'hydroxyde de guanidinium (" no-lye ") face à l'hydroxyde de sodium (" lye ") semble provenir d'une meilleure efficacité de défrisage et d'une meilleure tolérance cutanée. Cependant ces technologies mettant en oeuvre des bases de la famille des hydroxydes demeurent très agressives pour le cheveu et le cuir chevelu et demandent un contrôle très strict de la durée d'application pour éviter les irritations trop fortes et l'altération des cheveux pouvant aller jusqu'à la cassure. Cette agressivité provenant de la causticité des hydroxydes justifie que ces compositions pour la lanthionisation du cheveu ne soient pas actuellement utilisées pour la permanente (" hair waving ") mais réservées au défrisage (" hair straightening " ou " hair relaxing ").

De plus, les hydroxydes sont connus pour être de bons agents d'hydrolyse des fonctions amides (cf par exemple March's Advanced Organic Chemistry, 5ed., Wiley Interscience, New York, " Hydrolysis of Amides " pages 474 et suivantes) qui conduisent donc à la rupture des liaisons peptidiques par attaque nucléophile directe. Il est ainsi probable que les altérations constatées au niveau des cheveux et des matières kératiniques au sens large soient en grande partie dues à une hydrolyse partielle des liaisons amides de la kératine.

Il existe donc un réel besoin pour la mise en forme des cheveux de compositions moins agressives pour le cheveu et la peau.

Diverses études ont été conduites en vue de remédier à la fois aux inconvénients des agents réducteurs (première technique) et/ou des hydroxydes (deuxième technique).

Ainsi pour remplacer l'acide thioglycolique, de nombreux agents réducteurs ont été proposés, mais l'acide thioglycolique sous sa forme de thioglycolate d'ammonium reste à la fois le composé de référence et le plus largement utilisé dans les formulations cosmétiques, pour la mise en forme permanente des cheveux.

Toutefois, l'utilisation d'agents réducteurs conduit à une durabilité insuffisante pour la mise en forme permanente, ce qui oblige à répéter fréquemment le traitement et conduit inévitablement à des dégradations au fur et à mesure des superpositions. Par ailleurs, la causticité des hydroxydes limite leur utilisation au strict domaine du défrisage. Aucune étude n'a permis de proposer comme actif de mise en forme permanente des cheveux une base qui serait à la fois efficace et moins agressive que la soude ou les hydroxydes au sens large.

Après d'importantes études, on vient maintenant de découvrir, de façon tout à fait surprenante et inattendue, que l'on pouvait effectuer la première étape du procédé de lanthionisation avec des amines secondaires ou tertiaires. On obtient ainsi d'excellents résultats en terme d'intensité de mise en forme permanente, de qualités cosmétiques et mécaniques des cheveux, avec une durabilité supérieure à celle pouvant être obtenue avec le produit de référence pour la mise en forme permanente à savoir le thioglycolate d'ammonium.

La présente invention a donc pour objet une composition cosmétique comprenant, dans un milieu cosmétiquement acceptable, au moins une amine secondaire ou tertiaire, le milieu cosmétiquement acceptable et l'amine étant choisis de façon telle que l'amine secondaire ou tertiaire réagisse sur les cystines des fibres kératiniques, par une réaction de bêta-élimination produisant de la déhydro-alanine et conduisant à la formation de lanthionine, pour réaliser avec les fibres kératiniques des boucles de diamètre compris entre 0,2 et 3 cm, en moins de 60 minutes.

Avantageusement, la durée de la mise en forme permanente est inférieure à 50 minutes, et plus avantageusement inférieure à 40 minutes.

Par "amines secondaires ou tertiaires", utilisables à titre d'actifs de béta-élimination conduisant à la lanthionisation, on entend tout dérivé résultant de la substitution de deux ou trois atomes d'hydrogène de l'ammoniac par des radicaux carbonés simplement liés à l'atome d'azote ne contenant pas dans sa formule d'ions hydroxydes et capable d'accepter un proton. Ces amines secondaires ou tertiaires sont plus particulièrement sélectionnées dans le groupe des amines secondaires ou tertiaires cycliques et encore plus particulièrement dans le groupe des amines tertiaires cycliques.

Selon l'invention, on peut à titre d'exemple, citer comme amine tertiaire utilisable à titre d'actif de bêta-élimination conduisant à la lanthionisation, le 1,4,7-triméthyl-1,4,7-triazacyclononane (Registry Number [96556-05-7] et, comme amine secondaire utilisable à titre d'actif de bêta-élimination conduisant à la lanthionisation, le 1,4,7-triazacyclononane (Registry Number [4730-54-5].

Dans les compositions selon l'invention destinées à un processus de mise en forme permanente des fibres kératiniques, l'amine secondaire ou tertiaire est avantageusement présente à une concentration molaire comprise entre 0,01M et 4M, ce qui correspond à des concentrations comprises entre 0,1% et 80% en poids par rapport au poids total de la composition, et plus avantageusement, à une concentration comprise entre 0,05M et 2M, ce qui correspond à des concentrations comprises entre 0,5% et 40% en poids par rapport au poids total de la composition.

Le pH des compositions selon l'invention est, de préférence, compris entre 9,6 et 14, et plus particulièrement compris entre 11 et 13.

Avantageusement, dans les compositions de l'invention, l'amine secondaire ou tertiaire constitue le seul actif de mise en forme permanente.

Les compositions selon l'invention peuvent également contenir des agents réducteurs connus, tels que par exemple l'acide thioglycolique ou l'acide thiolactique et leurs dérivés esters et amides, notamment le monothioglycolate de glycérol, la cystéamine et ses dérivés acylés en C1-C4 tels que la N-acétyl-cystéamine ou la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides décrits dans la demande de brevet EP-A-354 835, les N-mono ou N,N-dialkylmercapto-4-butyramides décrits dans le demande de brevet EP-A-368 763, les aminomercaptoalkylamides, décrits dans le demande de brevet EP-A-432 000, les dérivés des acides N-(mercaptoalkyl)succinamiques et des N-(mercaptoalkyl)succinimides décrits dans le demande de brevet EP-A-465 342, les alkylamino mercaptoalkylamides décrits dans la demande de brevet EP-A-514 282, le mélange azéotrope de thioglycolate de 2-hydroxypropyle et de thioglycolate de (2-hydroxy-1-méthyl)éthyle décrits dans la demande de brevet FR-A-2 679 448, les mercaptoalkylaminoamides décrits dans la demande de brevet FR-A-2 692 481, les N-mercaptoalkylalcanediamides décrits dans le demande de brevet EP-A-653 202, ainsi que les dérivés d'acide formamidine sulfinique décrits dans la demande PCT/US01/43124, déposée par la Demanderesse.

Lorsque les compositions selon l'invention contiennent au moins un agent réducteur, celui-ci est avantageusement présent à une concentration maximale de 20% en poids, et de préférence, comprise entre 0,1 et 10% en poids par rapport au poids total de la composition.

Les compositions selon l'invention peuvent également contenir des hydroxydes connus choisis de préférence parmi les hydroxydes de métaux alcalins ou alcalino-terreux ou de métaux de transition ou organiques tels que l'hydroxyde de sodium, l'hydroxyde de potassium, l'hydroxyde de lithium, l'hydroxyde de rubidium, l'hydroxyde de césium, l'hydroxyde de francium, l'hydroxyde de béryllium, l'hydroxyde de magnésium, l'hydroxyde de calcium, l'hydroxyde de strontium, l'hydroxyde de barium, l'hydroxyde de molybdenium, l'hydroxyde de manganèse, l'hydroxyde de zinc, l'hydroxyde de cobalt, l'hydroxyde de cadmium, l'hydroxyde de cérium, l'hydroxyde de lanthanium, l'hydroxyde d'actinium, l'hydroxyde de thorium, l'hydroxyde d'aluminium, l'hydroxyde de guanidinium et les hydroxydes d'ammoniums quaternaires.

Lorsque les compositions de l'invention contiennent au moins un hydroxyde, celui-ci est avantageusement présent à une concentration allant de 0,01 % à 3,5 % en poids, et de préférence, comprise entre 0,05 et 1,5 % en poids par rapport au poids total de la composition.

Selon un mode de réalisation particulièrement avantageux des compositions selon l'invention, ces dernières contiennent 0 % de base appartenant à la famille des hydroxydes, choisie notamment parmi les hydroxydes de métaux alcalins ou alcalino-terreux ou de métaux de transition ou organiques.

Selon un mode de réalisation préféré, les compositions contiennent également un agent tensioactif de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque les compositions basiques contiennent au moins un agent tensioactif, celui-ci est généralement présent à une concentration maximale de 30 % en poids, et de préférence, comprise entre 0,5 et 10 % en poids par rapport au poids total de la composition.

Dans le but d'améliorer les propriétés cosmétiques des cheveux ou encore d'en atténuer ou d'éviter leur dégradation, la composition basique peut également contenir un agent traitant de nature cationique, anionique, non-ionique ou amphotère.

Parmi les agents traitants particulièrement préférés, on peut notamment citer ceux décrits dans les brevets français n° 2.598.613 et n° 2.470.596. On peut également utiliser comme agents traitants des silicones volatiles ou non, linéaires ou cycliques et leurs mélanges, les polydiméthylsiloxanes, les polyorganosiloxanes quatemisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n° 4.749.732, des polyorganosiloxanes tels que le copolymère polydiméthylsiloxane- polyoxyalkyle du type Diméthicone Copolyol, un polydiméthylsiloxane à groupements terminaux stéaroxy (stéaroxydiméthicone), un copolymère polydiméthylsiloxane- dialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

Les compositions basiques selon l'invention peuvent également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n°83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO2/PDM (polydiméthylsiloxane), le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylèneglycol, le monométhyléther de dipropylèneglycol, le monoéthyléther de l'éthylèneglycol et le monoéthyléther du diéthylèneglycol, des alcanediols en C3-C6 tels que par exemple le propanediol-1,2, le propanediol-1,3 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants ou complexants, des agents opacifiants, des filtres solaires ainsi que des parfums et des conservateurs.

La composition basique selon l'invention se présente essentiellement sous forme aqueuse, notamment sous la forme d'une lotion épaissie ou non, d'une crème ou d'un gel.

La composition basique selon l'invention peut également contenir un solvant tel que par exemple de l'éthanol, du propanol ou de l'isopropanol ou du butanol ou encore du glycérol, à une concentration maximale de 20 % par rapport au poids total de la composition.

Le milieu cosmétiquement acceptable des compositions basiques selon l'invention est, de préférence, de l'eau ou une solution hydroalcoolique d'un alcool en C2-C8, tel que l'éthanol ou l'isopropanol.

Les compositions selon l'invention peuvent également se présenter sous forme d'une crème épaissie de façon à maintenir les cheveux dans la forme finale souhaitée. On réalise ces crèmes, sous forme d'émulsions "lourdes", par exemple à base de stéarate de glycéryle, de stéarate de glycol, de cires auto-émulsionnables ou d'alcools gras.

On peut également utiliser des liquides ou des gels contenant des agents épaississants tels que des polymères ou des copolymères carboxyvinyliques qui "collent" les cheveux et les maintiennent dans la forme souhaitée pendant le temps de pose.

Les compositions selon l'invention peuvent contenir, en outre, au moins un adjuvant choisi parmi les silicones sous forme soluble, dispersée, micro-dispersée, les agents tensio-actifs non-ioniques, anioniques, cationiques et amphotères, les céramides, les glycocéramides et pseudo-céramides, les vitamines et pro-vitamines dont le panthénol, les huiles végétales, animales, minérales et synthétiques, les cires autres que les céramides, les glycocéramides et pseudo-céramides, les filtres solaires hydrosolubles et liposolubles, siliconés ou non siliconés, les agents nacrants et opacifiants, les agents séquestrants, les agents plastifiants, les agents solubilisants, les agents acidifiants, les agents épaississants minéraux et organiques, les agents anti-oxydants, les hydroxyacides, les agents de pénétration, les parfums et les agents conservateurs.

L'invention concerne également un kit comprenant au moins deux compartiments, l'un des compartiments (i) comprenant, dans un milieu cosmétiquement acceptable, au moins une amine secondaire ou tertiaire apte à réagir sur les cystines des fibres kératiniques, par une réaction de bêta-élimination produisant de la déhydro-alanine et conduisant à la formation de lanthionine, pour réaliser avec les fibres kératiniques des boucles de diamètre compris entre 0,2 et 3 cm, en moins de 60 minutes.

De préférence, le kit selon l'invention, comprend, en outre, une composition supplémentaire (ii) de soin, de conditionnement, de maquillage, de démaquillage, de protection, de nettoyage ou de lavage des fibres kératiniques.

Les compositions des kits selon l'invention sont conditionnées dans des compartiments ou récipients ou dispositifs distincts, accompagnés, éventuellement, de moyens d'application appropriés, identiques ou différents, tels que les pinceaux, les brosses, les éponges.

Un autre objet de l'invention concerne un procédé de mise en forme permanente des matières kératiniques mettant en oeuvre une composition cosmétique comprenant, dans un milieu cosmétiquement acceptable, au moins une amine secondaire ou tertiaire, le milieu cosmétiquement acceptable et l'amine étant choisis de façon telle que l'amine secondaire ou tertiaire réagisse sur les cystines des fibres kératiniques, par une réaction de bêta-élimination produisant de la déhydro-alanine et conduisant à la formation de lanthionine, pour réaliser avec les fibres kératiniques des boucles de diamètre compris entre 0,2 et 3 cm, en moins de 60 minutes.

Avantageusement, dans le procédé selon l'invention, la durée de la mise en forme permanente est inférieure à 50minutes, et plus avantageusement inférieure à 40 minutes

Dans le procédé de mise en forme permanente selon l'invention, on applique, avantageusement, la composition selon l'invention sur des cheveux secs ou préalablement mouillés, préalablement enroulés sur des rouleaux ou des bigoudis, ayant un diamètre de 2 à 30 mm, la composition pouvant être appliquée au fur et à mesure de l'enroulage des cheveux; on laisse ensuite agir la composition pendant une durée allant de 5 à 60 minutes, de préférence 5 à 40 minutes. Après avoir enlevé les rouleaux, on rince abondamment les cheveux.

Selon la présente invention, on peut, avantageusement, après application de la composition selon l'invention, soumettre la chevelure à un traitement thermique par chauffage à une température comprise entre 30 et 60°C. Dans la pratique, cette opération peut être conduite au moyen d'un casque de coiffure, d'un sèche-cheveux, d'un dispensateur de rayons infrarouges et d'autres appareils chauffants classiques.

On peut utiliser, à la fois comme moyen de chauffage et de mise en forme de la chevelure, un fer chauffant à une température comprise entre 60 et 220°C et de préférence entre 120 et 200°C.

Encore un autre objet de l'invention concerne l'utilisation d'une amine secondaire ou tertiaire comme agent actif de mise en forme permanente des fibres kératiniques.

L'invention concerne aussi un agent actif de mise en forme permanente des fibres kératiniques, au moyen d'une réaction de béta-élimination produisant de la déhydro-alanine et conduisant à la formation de lanthionine, comprenant au moins une amine secondaire ou tertiaire.

L'invention pourra être mieux comprise à l'aide des exemples non limitatifs qui suivent et qui constituent des modes de réalisation préférentiels des compositions selon l'invention.

### EXEMPLE 1:

On réalise une composition de permanente simplifiée contenant le 1,4,7-triméthyl-1,4,7-triazacyclononane à une concentration de 1M dans l'eau, en tant qu'actif de permanente. Le pH de la composition est de 13,0. On applique cette composition sur des cheveux caucasiens, préalablement enroulés sur des bigoudis, pendant 30 minutes à une température de 50°C. On rince et on sèche. Les cheveux présentent de belles boucles et sont doux au toucher.

### EXEMPLE 2:

On réalise une composition de permanente simplifiée contenant le 1,4,7-triméthyl-1,4,7-triazacyclononane à une concentration de 0,1M dans l'eau, en tant qu'actif de permanente. Le pH de la composition est de 12,5. On applique cette composition sur des cheveux caucasiens, préalablement enroulés sur des bigoudis, pendant 40 minutes à une température de 50°C. On rince et on sèche. Les cheveux présentent de belles boucles et sont doux au toucher.

### EXEMPLE 3:

On réalise une composition de permanente simplifiée contenant le 1,4,7-triazacyclononane à une concentration de 0,2 M dans l'eau, en tant qu'actif de permanente. Le pH de la composition est de 12,1. On applique cette composition sur des cheveux caucasiens, préalablement enroulés sur des bigoudis, pendant 40 minutes à une température de 50°C. On rince et on sèche. Les cheveux présentent de belles boucles et sont doux au toucher.

### EXEMPLE 4:

On réalise une composition de permanente simplifiée contenant le 1,4,7-triazacyclononane à une concentration de 2M dans l'eau, en tant qu'actif de permanente. Le pH de la composition est de12,8. On applique cette composition sur des cheveux caucasiens, préalablement enroulés sur des bigoudis, pendant 30 minutes à une température de 50°C. On rince et on sèche. Les cheveux présentent de belles boucles et sont doux au toucher.

## Revendications

1. Composition cosmétique comprenant, dans un milieu cosmétiquement acceptable, au moins une amine secondaire ou tertiaire, le milieu cosmétiquement acceptable et l'amine étant choisis de façon telle que l'amine secondaire ou tertiaire réagisse sur les cystines des fibres kératiniques, par une réaction de bêta-élimination produisant de la déhydro-alanine et conduisant à la formation de lanthionine, pour réaliser avec les fibres kératiniques des boucles de diamètre compris entre 0,2 et 3 cm, en moins de 60 minutes.

2. Composition selon la revendication 1, **caractérisée par** le fait la durée de la mise en forme permanente est inférieure à 50 minutes, et plus avantageusement inférieure à 40 minutes.

3. Composition selon l'une quelconque des revendications précédentes, **caractérisée par le fait que** l'amine secondaire ou tertiaire est présente à une concentration molaire comprise entre 0,01M et 4M, et plus avantageusement, à une concentration comprise entre 0,05M et 2M.

4. Composition selon l'une quelconque des revendications précédentes, **caractérisée par le fait que** le pH est compris entre 9,6 et 14, et plus particulièrement compris entre 11 et 13.

5. Composition selon l'une quelconque des revendications précédentes, **caractérisée par le fait qu'**elle contient 0 % de base appartenant à la famille des hydroxydes.

6. Composition selon l'une quelconque des revendications précédentes, **caractérisée par le fait qu'**elle contient, en outre, au moins un adjuvant choisi parmi les silicones sous forme soluble, dispersée, micro-dispersée, les agents tensio-actifs non-ioniques, anioniques, cationiques et amphotères, les céramides, les glycocéramides et pseudo-céramides, les vitamines et pro-vitamines dont le panthénol, les huiles végétales, animales, minérales et synthétiques, les cires autres que les céramides, les glycocéramides et pseudo-céramides, les filtres solaires hydrosolubles et liposolubles, siliconés ou non siliconés, les agents nacrants et opacifiants, les agents séquestrants, les agents plastifiants, les agents solubilisants, les agents acidifiants, les agents épaississants minéraux et organiques, les agents anti-oxydants, les hydroxyacides, les agents de pénétration, les parfums et les agents conservateurs.

7. Kit comprenant au moins deux compartiments, l'un des compartiments (i) comprenant, dans un milieu cosmétiquement acceptable, au moins une amine secondaire ou tertiaire apte à réagir sur les cystines des fibres kératiniques, par une réaction de bêta-élimination produisant de la déhydro-alanine et conduisant à la formation de lanthionine, pour réaliser avec les fibres kératiniques des boucles de diamètre compris entre 0,2 et 3 cm, en moins de 60 minutes.

8. Kit selon l'une quelconque des revendications précédentes, **caractérisé par le fait qu'**il comprend, en outre, une composition supplémentaire (ii) de soin, de conditionnement, de maquillage, de démaquillage, de protection, de nettoyage ou de lavage des fibres kératiniques.

9. Procédé de mise en forme permanente des matières kératiniques mettant en oeuvre une composition cosmétique comprenant, dans un milieu cosmétiquement acceptable, au moins une amine secondaire ou tertiaire, le milieu cosmétiquement acceptable et l'amine étant choisis de façon telle que l'amine réagisse sur les cystines des fibres kératiniques, par une réaction de bêta-élimination produisant de la déhydro-alanine et conduisant à la formation de lanthionine, pour réaliser avec les fibres kératiniques des boucles de diamètre compris entre 0,2 et 3 cm, en moins de 60 minutes.

10. Procédé de mise en forme permanente des matières kératiniques selon la revendication 9, **caractérisé par le fait qu'**après application de la composition, on soumet la chevelure à un traitement thermique par chauffage à une température comprise entre 30 et 60°C.

11. Procédé de mise en forme permanente des matières kératiniques selon la revendication 9 ou 10, **caractérisé par le fait qu'**on utilise, comme moyen de chauffage de la chevelure, un fer chauffant à une température comprise entre 60 et 220°C et de préférence entre 120 et 200°C.

12. Procédé de mise en forme permanente des matières kératiniques selon la revendication 9 à 11, **caractérisé par le fait que** la durée de la mise en forme permanente est inférieure à 50 minutes, et plus avantageusement inférieure à 40 minutes

13. Utilisation d'une amine secondaire ou tertiaire comme agent actif de mise en forme permanente des fibres kératiniques.

14. Agent actif de mise en forme permanente des fibres kératiniques, au moyen d'une réaction de béta-élimination produisant de la déhydro-alanine et conduisant à la formation de lanthionine, comprenant au moins une amine secondaire ou tertiaire.
